# EUROPEAN PATENT SPECIFICATION

(11) **EP 1 005 900 B1**
(45) Date of publication and mention of the grant of the patent: **04.05.2005**
(21) Application number: 00101313.5
(22) Date of filing: 12.11.1996
(51) Int. Cl.: B01F 13/00, B01F 13/06, A61F 2/46

(54) **Method and device for introducing a liquid component for bone cement into a mixing vessel**
Verfahren und Vorrichtung zum Zuführen eines Flüssigen Bestandteiles für Knochenzement in einer Mischvorrichtung
Procédé et dispositif d'alimentation d'un composant liquide pour un ciment osseux dans un récipient de mélange

(30) Priority: 13.11.1995 US 545591; 22.10.1996 US 734817
(43) Date of publication of application: 07.06.2000
(62) Divisional of application: 96939409.7
(73) Proprietor: CEMVAC SYSTEM AKTIEBOLAG, 589 41 Linköping (SE)
(72) Inventor: Jonsson, Sören, S-589 41 Linköping (SE)

(56) References cited:
- WO-A-94/26402
- DE-A- 2 921 565
- US-A- 5 306 277

## Description

### BACKGROUND OF THE INVENTION

### Field of the Invention

The present invention relates to a method for introducing into a mixing vessel under partial vacuum a liquid component of bone cement to be mixed with a powder component of bone cement. The invention also relates to an apparatus for introducing into a mixing vessel under partial vacuum a liquid component of bone cement to be mixed with a powder component.

### Description of the Prior Art

Bone cement is prepared by mixing polymethyl methacrylate in powder form, with liquid monomethyl methacrylate in a mixing container. Both the liquid component and the combined mixture give off substances in gaseous form which are environmentally harmful and injurious to the health. For this reason, it is important for the introduction of the bone cement components into the mixing container and the mixing process itself, to take place in such a way that the smallest possible quantity of the harmful gases escape into the surrounding environment. Mixing vessels in which the introduced components were successfully prepared into bone cement without a substantial release of the aforementioned gases are described in SE-C-8901599-4 and SE-A0-9201353-1 and WO 94/26403, for example. In order for the bone cement to develop optimal strength during use it is also important for the components comprising the cement to have well-mixed, predetermined portions.

In said WO-publication a glass ampoule containing a liquid component of a bone cement is surrounded by a container which is in reclosable communication with the atmosphere. A second container surrounds the first container so that when the mixing vessel is opened, the contents of the ampoule, under the effect of a partial vacuum inside the mixing vessel, can be sucked down into it, a space formed between the aforementioned inner container and outer container is filled with a second bone cement component in powder form which is caused by displacement of the inner container relative to the outer container, to move from a first position in which the space does not communicate with the atmosphere or the mixing vessel to a second position in which the space communicates with the atmosphere and the mixing vessel, so that the powdered bone cement component is sucked down into the mixing vessel under the effect of the partial vacuum inside it.

A device for carrying out the above method which device is also explained in said WO-publication is characterized in that it comprises, an inner container communicating with the atmosphere, and is so arranged as to enclose the glass ampoule containing the liquid bone cement component, and to communicate with the aforementioned mixing vessel, and which includes means for opening the ampoule so that its contents, under the effect of the partial vacuum inside the mixing vessel, can be sucked down into it. The outer container at least partially encloses the inner container and is also arranged so as to communicate with the mixing vessel and together with the inner container, defines a space therebetween which is filled with a certain quantity of the powdered component of bone cement. The inner container is capable of displacement from a first position to a second position, the first position characterized by the inner container preventing communication between both the mixing vessel and the atmosphere, and the second position characterized, in which communication between the mixing vessel on the one hand and the atmosphere on the other hand is open, so that the bone cement component in powder form, under the effect of the partial vacuum inside the mixing vessel, can be sucked into it without escape of gases.

### SUMMARY OF THE INVENTION

One object of the present invention is to make available a method and device for introducing into a mixing vessel the liquid component of bone cement which avoids the risk of gas release when feeding said bone cement components into the mixing vessel. Another object is to make the mixing as convenient and simple as possible for a person using the device. Still another object is to make the mixing as safe as possible for said person i.e. reduce to a minimum the failure rate of mixing. This is achieved in accordance with the method and apparatus of the invention.

The invention is definied by the appended independent claims. Embodiments are set forth in the following description and in the drawings.
Figure 1 illustrates an embodiment of a mixing vessel in which the powder form of the bone cement pre-exists within the mixing vessel;
Figure 2 illustrates a cassette according to the invention that houses a glass ampoule which contains the liquid form of the bone cement;
Figure 3 illustrates a cross-sectional view of the cassette of Figure 1 connected to a mixing vessel pre-filled with powder form of the bone cement prior to mixing the cement components;
Figure 4 illustrates the mixing vessel of Figure 2, feeding the liquid into the vessel.

### DETAILED DESCRIPTION OF THE INVENTION

The designations 1 and 2 are used generally in the drawing in respect of a feed arrangement and a mixing vessel. The latter comprises an interior 2a, a cylindrical container 3 comprised of an outer cylinder wall 3a, a bottom 4 at one end of the container and a spout 5 with sealed opening at the other end, together with an agitator 6, received within said spout and mixing vessel and capable of axial, vertical movement inside the container 3.The agitator 6 consists of an agitator disc 6a attached to a tubular agitator rod 6b. The agitator 6 is mounted so that it is free to vertically slide up and down while maintaining a seal in the spout 5, in such a way that the plurality of holes 6h in agitator disk 6a can be used to bring about through mixing of the bone cement components A, B within mixing vessel 2 with no gaseous escape to atmosphere. Once mixing is complete, and once a lock 7 has been removed, the bottom 4 can be axially displaced inside the cylinder by upward movement of piston head 80, moving towards the spout 5. The pistonlike function of bottom 4, upwardly pushes and then discharges the mixed bone cement via the hollow agitator rod 6b, which now serves as a discharge nozzle. The interior of the container 2 communicates via a filter 8 with a vacuum source (not shown) during feeding and mixing of the bone cement components A, B. Rapid and effective feeding of the bone cement components into the mixing vessel, and safe handling of the gases that are environmentally harmful and injurious to human health, are achieved in this way.

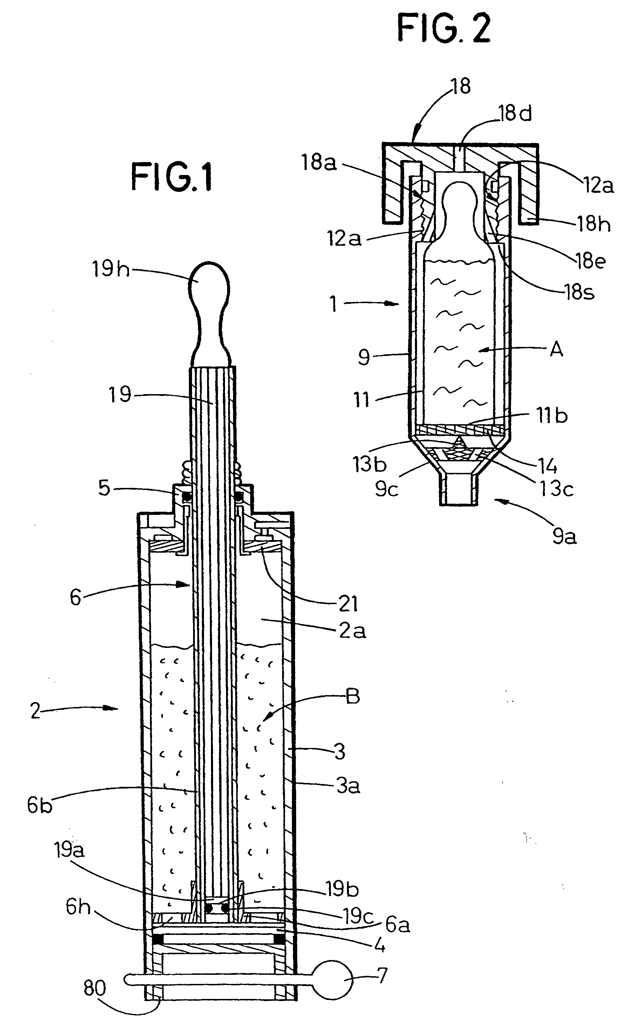

Turning attention now to Figures 1-4, the present invention will now be described. The powdered component B of bone cement pre-exists within the mixing vessel 2 prior to any mixing procedures, and the container 9 contains only the liquid component A. It will become clearer after reading the following description that the main characteristic of the embodiment is that the liquid component A will be drawn into the mixing vessel under vacuum, and that an ampoule arrangement is provided wherein the ampoule rests on the mixing vessel and wherein the contents feed downward through the tubular agitator rod 6b, and enter vessel 2 in the vicinity of the vessel bottom 4.

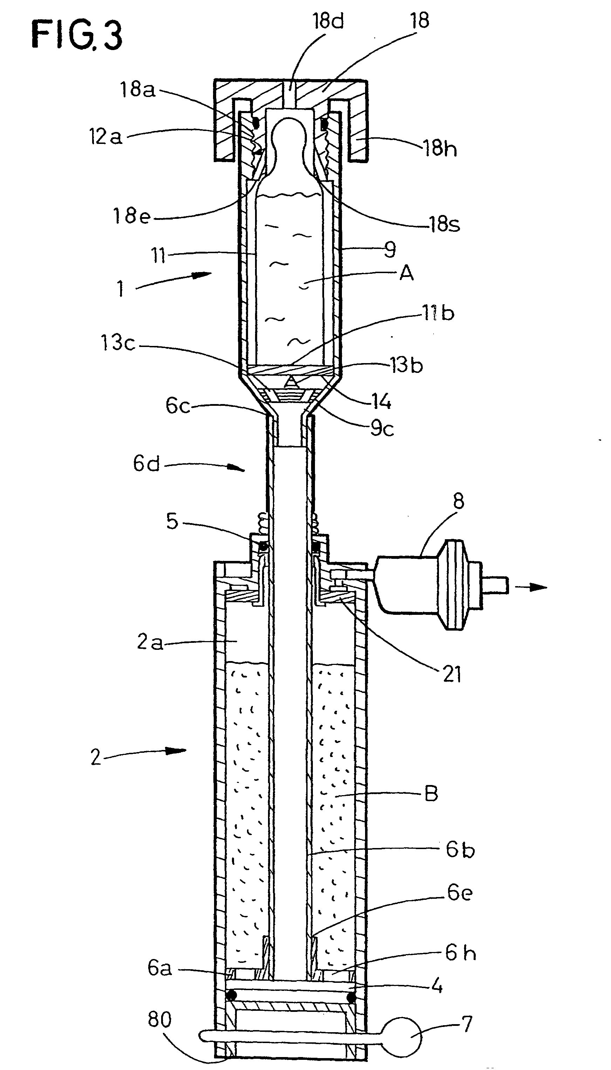

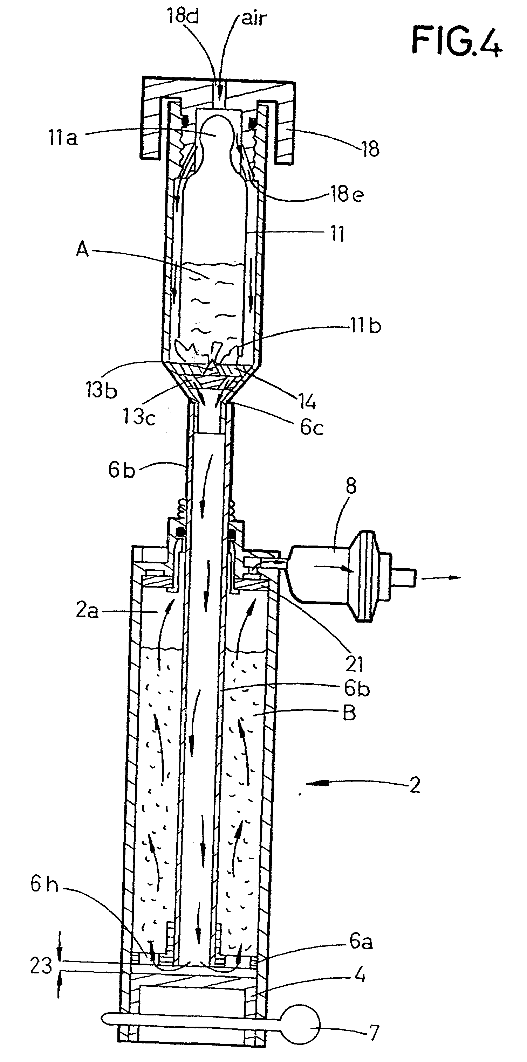

In accordance with the embodiment of Figures 1-4 mixing vessel 2 is shown as being pre-filled with the powder component B of the bone cement. A tightening rod 19 is received within tubular agitator rod 6b of agitator 6 and has plug 19a and O-ring 19c inserted within a groove 19b thereof, to form an air-tight seal so that powdered contents B are not contacted by and affected by atmospheric air which is capable of downwardly travelling along tubular rod 6b to vessel bottom 4. Just prior to introducing glass ampoule 11 on top of mixing vessel 2, tightening rod 19 is completely removed from tube 6b, wherein the cylindrical container 9 is placed on top of vessel 2 by inserting funnel-shaped neck 9c into mouth 6c at the first end 6d of tubular rod 6b. Figure 2 shows in greater detail that glass ampoule tip 11a is pointing upwards when inserted within container 9, and that the ampoule is resting upon the upward cone 13b of breaking means 13, said means having internal passages 13c for allowing liquid there through once it passes filter 14. Figure 2 also illustrates that cap 18 has gripping means 18h for facilitating the operative threading movement of cap 18 along container threads 12a. Figure 3 shows the ampoule 11 just prior to being broken. Figure 4 shows that when handle 18h of the cap is turned so as to downwardly displace the cap 18 through action of the interacting threads 12a and 18a, shoulder 18s pushed downwardly against ampoule 11, causing upward-facing, pointed cone 13b to break bottom 11b of the ampoule, thereby allowing liquid contents A to flow through filter 14 under suction downwardly into hollow tube 6b as previously described. As Figure 4 shows, openings 18d and 18e in cap 18, allow atmospheric air to be communicated into the interior of container 9 under suction, also as previously described, thereby preventing noxious fumes escaping to atmosphere. A small gap 23 exists between vessel bottom 4 and agitator disk 6a so that as liquid A descends tubular rod 6b, exists open end 6e, then it enters gap 23, which behaves as a passage for percolating an air/liquid mixture upwardly through holes 6h in the agitator disk 6a, so that air bubbles cause liquid A to thoroughly mix with the powder component B, while under the continuing action of the vacuum source.

It should be obvious that the container 9 according to the present invention can be used for any kind of mixing vessel 2 e.g. as shown in various embodiments in WO 97/18031.

## Claims

1. A method for introducing into a mixing vessel (2) under partial vacuum a liquid component (A) of bone cement to be mixed with a powder component (B) of bone cement, the method comprising the steps of providing a container (9) which has an open interior, a threadable cap (18) and means (13) for breaking a glass ampoule, connecting said container in an airtight manner to the mixing vessel (2), placing a glass ampoule (11) containing said liquid component (A) into said open interior, turning said cap for pushing downwards on said ampoule (11) for breaking it against said breaking means (13b) allowing said container (9) to feed the liquid component (A) through an opening (13c) in the container into said mixing vessel (2), whereby ambient air is allowed to enter into said open interior of the container (9) through an opening (18d) in the cap of the container, **characterized by** feeding only said liquid component (B) from said container (9) into said mixing vessel (2), said mixing vessel (2) being pre-filled with said powder component (B) of said bone cement
allowing said liquid component (A) to enter said mixing vessel in the vicinity of a vessel bottom (4), and
allowing said liquid component (A), mixed with said ambient air, to percolate upwardly, so that air bubbles cause said liquid component (A) to thoroughly mix with said powder component (B).

2. An apparatus for introducing into a mixing vessel (2) under partial vacuum a liquid component (A) of bone cement to be mixed with a powder component (B), the apparatus comprising a container (9) which has an open interior for receiving at least one glass ampoule (11) containing said liquid component and a threadable cap (18) for pushing downwards on said ampoule (11) said interior including a means (13b) for breaking said ampoule (11) when said cap pushes on said ampoule (11) thereby allowing said container (9) to feed the liquid component (A) through an opening in the container, into said mixing vessel (2), the container opening being arranged for air-tight connection to the mixing vessel, and the cap (18) of the container having an opening (18d) for allowing ambient air to enter into said open interior of the container (9), **characterized by** said container being adapted for feeding only said liquid component (A) into said mixing vessel (2), said mixing vessel being pre-filled with said powder component (B) of said bone cement
said container is connectable to said mixing vessel in the vicinity of a vessel bottom (4), such that said liquid component (A) is introducible into said mixing vessel in the vicinity of said vessel bottom (4),
for allowing said liquid component (A), to mix with said ambient air, and to percolate upwardly, so that air bubbles cause said liquid component (A) to thoroughly mix with said powder component (B).

## Patentansprüche

1. Verfahren zum Einleiten einer flüssigen Komponente (A) eines Knochenzements in ein Mischgefäß (2) unter Teilvakuum, um mit einer Pulverkomponente (B) des Knochenzements gemischt zu werden, wobei das Verfahren die Schritte umfasst:
Bereitstellen eines Behälters (9), welcher ein offenes Inneres, eine mit einem Gewinde ausgebildete Kappe (18) und Mittel (13) zum Zerbrechen einer Glasampulle aufweist, Verbinden des Behälters mit dem Mischgefäß (2) auf luftdichte Weise,
Platzieren einer Glasampulle (1), welche die flüssige Komponente (A) enthält, in dem offenen Inneren,
Drehen der Kappe, um abwärts auf die Ampulle (11) zu drücken, um diese gegen das Zerbrechmittel (13b) zu zerbrechen,
Ermöglichen, dass der Behälter (9) die flüssige Komponente (A) durch eine Öffnung (13c) in dem Behälter in das Mischgefäß (2) speist, wobei es der umgebenden Luft ermöglicht wird in das offene Innere des Behälters (9) durch eine Öffnung (18d) in der Kappe des Behälters zu gelangen, **gekennzeichnet durch**,
Füllen nur der flüssigen Komponente (B) von dem Behälter (9) in das Mischgefäß (2), wobei das Mischgefäß (2) vorgefüllt ist mit der Pulverkomponente (B) des Knochenzements, Ermöglichen, dass die flüssige Komponente (A) in das Mischgefäß in der Nähe von einem Gefäßboden (4) gelangt und
Ermöglichen, dass die flüssige Komponente (A), welche mit der umgebenden Luft gemischt ist, aufwärts perkoliert, so dass Luftblasen bewirken, dass die flüssige Komponente (A) sich gründlich mit der Pulverkomponente (B) mischt.

2. Vorrichtung zum Einleiten einer flüssigen Komponente (A) eines Knochenzements in ein Mischgefäß (2) unter Teilvakuum, um mit einer pulverförmigen Komponente (B) gemischt zu werden, wobei die Vorrichtung umfasst:
einen Behälter (9), welcher ein offenes Inneres zur Aufnahme von zumindest einer Glasampulle (11), welche die flüssige Komponente enthält und
eine mit einem Gewinde ausgestattete Kappe (18), um auf die Ampulle (11) abwärts zu drücken aufweist, wobei das Innere Mittel (13b) zum Zerbrechen der Ampulle ( 11 ) aufweist, wenn die Kappe auf die Ampulle (11) drückt, wodurch es dem Behälter (9) ermöglicht wird, die flüssige Komponente (A) durch eine Öffnung in dem Behälter in das Mischgefäß (2) zu speisen, wobei die Behälteröffnung für eine luftdichte Verbindung mit dem Mischgefäß ausgebildet ist und die Kappe (18) des Behälters eine Öffnung (18d) aufweist, um es der umgebenden Luft zu ermöglichen, in das offene Innere des Behälters (9) zu gelangen, **dadurch gekennzeichnet,**
**dass** der Behälter ausgebildet ist, um nur die flüssige Komponente (A) in das Mischgefäß (2) zu speisen, wobei das Mischgefäß vorgefüllt ist mit der Pulverkomponente (B) des Knochenzements, wobei der Behälter verbindbar mit dem Mischgefäß in der Nähe des Gefäßbodens (4) ausgebildet ist, so dass die flüssige Komponente (A) einleitbar in das Mischgefäß in der Nähe des Gefäßbodens ist, um es der flüssigen Komponente (A) zu ermöglichen, sich mit der umgebenden Luft zu mischen und aufwärts zu perkolieren, so dass Luftblasen verursachen, dass die flüssige Komponente (A) sich gründlich mit der Pulverkomponente (B) mischt.

## Revendications

1. Procédé destiné à introduire à l'intérieur d'un récipient de mélange (2) sous un vide partiel un composant liquide (A) de ciment osseux à mélanger à un composant poudreux (B) de ciment osseux, le procédé comprenant les étapes consistant à fournir un réservoir (9) qui présente un intérieur ouvert, un capuchon qui peut être fileté (18) et des moyens (13) destinés à briser une ampoule en verre, raccorder ledit réservoir d'une manière étanche à l'air au récipient de mélange (2), placer une ampoule en verre (11) contenant ledit composant liquide (A) dans ledit intérieur ouvert, tourner ledit capuchon afin d'effectuer une poussée vers le bas sur ladite ampoule (11) pour la briser contre lesdits moyens de rupture (13b), permettre au dit récipient (9) d'amener le composant liquide (11) à travers une ouverture (13c) dans le réservoir à l'intérieur dudit réservoir de mélange (2), l'air ambiant étant autorisé à pénétrer dans ledit intérieur ouvert du réservoir (9) à travers une ouverture (18d) dans le capuchon du réservoir, **caractérisée par** l'amenée seule dudit composant liquide (B) depuis ledit réservoir (9) à l'intérieur dudit récipient de mélange (2), ledit récipient de mélange (2) étant pré-rempli dudit composant poudreux (B) dudit ciment osseux,
permettant au dit composant liquide (A) de pénétrer à l'intérieur dudit récipient de mélange au voisinage d'un fond (4) du récipient, et
permettant au dit composant liquide (A), mélangé au dit air ambiant, de percoler vers le haut, de sorte que des bulles d'air amènent ledit composant liquide (A) à se mélanger complètement au dit composant poudreux (B).

2. Appareil destiné à introduire à l'intérieur d'un récipient de mélange (2) sous un vide partiel un composant liquide (A) de ciment osseux à mélanger à un composant poudreux (B), l'appareil comprenant un réservoir (9) qui présente un intérieur ouvert destiné à recevoir au moins une ampoule en verre (11) contenant ledit composant liquide et un capuchon qui peut être fileté (18) destiné à effectuer une poussée vers le bas sur ladite ampoule (11), ledit intérieur comprenant des moyens (13b) destinés à briser ladite ampoule (11) lorsque ledit capuchon effectue une poussée sur ladite ampoule (11), permettant de ce fait au dit réservoir (9) d'amener le composant liquide (A) à travers une ouverture dans le réservoir à l'intérieur dudit récipient de mélange (2), l'ouverture du réservoir étant agencée en vue d'un raccordement étanche à l'air au récipient de mélange, et le capuchon (18) du réservoir présentant une ouverture (18d) destinée à permettre à l'air ambiant de pénétrer dans ledit intérieur ouvert du réservoir (9), **caractérisé en ce que** ledit réservoir est adapté pour n'amener que ledit composant liquide (A) à l'intérieur dudit récipient de mélange (2), ledit récipient de mélange étant pré-rempli dudit composant poudreux (B) dudit ciment osseux,
ledit réservoir pouvant être raccordé au dit récipient de mélange au voisinage d'un fond (4) du récipient, de telle sorte que ledit composant liquide (A) peut être introduit à l'intérieur dudit récipient de mélange au voisinage dudit fond (4) du récipient,
pour permettre au dit composant liquide (A) de se mélanger au dit air ambiant, et de percoler vers le haut, de sorte que des bulles d'air amènent ledit composant liquide (A) à se mélanger complètement au dit composant poudreux (B).
